# EUROPÄISCHE PATENTSCHRIFT

(11) **EP 1 170 590 B1**
(45) Veröffentlichungstag und Bekanntmachung des Hinweises auf die Patenterteilung: **05.04.2006**
(21) Anmeldenummer: 01115982.9
(22) Anmeldetag: 30.06.2001
(51) Int. Cl.: G01N 33/52, G01N 33/66, G01N 33/96

(54) **Adsorptionsmittelhaltige Kontrollflüssigkeit**
Control solution containing an adsorbent
Solution étalon contenant un adsorbent

(30) Priorität: 03.07.2000 DE 10032290
(43) Veröffentlichungstag der Anmeldung: 09.01.2002
(73) Patentinhaber: Roche Diagnostics GmbH, 68305 Mannheim (DE); F. HOFFMANN-LA ROCHE AG, 4070 Basel (CH)
(72) Erfinder: Knappe, Wolfgang-Reinhold, Dr., 67071 Ludwigshafen (DE); Gaa, Otto, Dr., 67551 Worms (DE); Zimmer, Volker, 69221 Dossenheim (DE); Hoenes, Joachim, Dr., 64673 Zwingenberg (DE); Hiller, Bernd, 68623 Lampertheim (DE); Wittmann, Franz, 68766 Hockenheim (DE); Koschorreck, Beate, 69198 Schriesheim (DE)

(56) Entgegenhaltungen:
- EP-B- 0 460 896
- US-A- 5 187 100
- US-A- 5 605 837
- US-A- 5 679 573

## Beschreibung

Die Erfindung betrifft eine Kontrollflüssigkeit zur Qualitäts- und/oder Funktionskontrolle eines Messsystems enthaltend Teststreifen und Messgerät für die Bestimmung von Analyten in flüssigen Proben.

Die Kontrolle von Körperfunktionen anhand der Bestimmung des Gehalts von einzelnen, meist niedermolekularen Metaboliten des Stoffwechsels in Körperflüssigkeiten ist ein unverzichtbares Instrument der modernen Medizin geworden. Prominente Beispiele sind die Blutzuckerselbstkontrolle bei Diabetikern und in neuerer Zeit verstärkt die Messung des Blutcholesteringehalts und der Lactatkonzentration im Blut, wobei letztere insbesondere in der Sportmedizin zur Überprüfung der individuellen Fitness Beachtung findet.

Für die zuverlässige, schnelle und unkomplizierte Analyse von Körperflüssigkeiten, insbesondere von Blut und Urin, haben sich eine Vielzahl von trägergebundenen Testelementen, sogenannten Teststreifen, etabliert. Einfache Teststreifen erlauben die visuelle Konzentrationsbestimmung des interessierenden Analyten, beispielsweise durch Farbveränderungen einer Reagenzschicht auf dem Teststreifen und Vergleich mit einer Farbskala, mit der wiederum Analytkonzentrationen korreliert sind. Komfortabler sind Messsysteme, die sowohl Teststreifen als auch Messgeräte enthalten. Diese Systeme erfassen - meist photometrisch oder elektrochemisch - die Veränderungen, die sich durch die Reaktion des Analyten mit den Reagenzien, die auf oder in dem Teststreifen enthalten sind, ergeben.

Da Teststreifen nicht in hundertprozentig identischen Chargen hergestellt werden können, ist es erforderlich, chargenspezifische Kalibrierungen der Messgeräte vorzunehmen. Dies geschieht heute vorzugsweise automatisch über chargenspezifische Codes, die vom Messgerät eingelesen oder vom Benutzer in das Messgerät eingegeben werden und die zu einer automatischen Anpassung eines Messwerteauswertealgorithmus führen.

Neben den fertigungsbedingten, chargenspezifischen Unterschieden unterliegen aber sowohl Teststreifen als auch Messgeräte Schwankungen in ihrer Messgenauigkeit und Zuverlässigkeit, die beispielsweise durch lange oder unsachgemäße Lagerung der Teststreifen verursacht sein können oder bei Messgeräten benutzungsbedingt entstehen können. Deshalb ist es erforderlich, in regelmäßigen Abständen eine Funktions- und Qualitätskontrolle des Messsystems durchzuführen, um Fehler rechtzeitig erkennen und gegebenenfalls beheben zu können. Zu diesem Zweck werden von den Herstellern der Messsysteme Kontrollflüssigkeiten (Synonym: Kontrolllösungen) angeboten, die jeweils für ein Messsystem spezifisch sind.

Die Qualitäts- und/oder Funktionskontrolle eines analytischen Systems, das aus Teststreifen und Messgerät besteht, soll gewährleisten, daß Messergebnisse, die bei einer Messung mit dem analytischen System erhalten werden, stets richtig, genau und wiederholbar sind. Insbesondere für die medizinische Diagnostik, die dem Arzt Anhaltspunkte für eine gezielte Therapie geben sollte, sind dies unabdingbare Voraussetzungen, um Fehldiagnosen oder -therapien zuverlässig auszuschließen.

Die Kontrollflüssigkeiten bestehen meist im wesentlichen aus wäßrigen, gepufferten Lösungen des Analyten in bekannter, vorbestimmter Konzentration. Sie können jedoch auch weitere Zusatzstoffe enthalten, die beispielsweise die Viskosität oder die Färbung der eigentlichen Probenflüssigkeit möglichst genau imitieren, mit dem Zweck, möglichst realistische Messbedingungen zu simulieren. Solche Kontrollflüssigkeiten sind beispielsweise aus US 3,920,580 und EP-B 0 514 485 bekannt.

In US 3,920,580 ist eine Kontrollflüssigkeit beschrieben, in der sogenannte "anti-diffusing agents", wie z. B. Rinderserumalbumin, Dextran und ähnliches, die Kontrolllösung blutähnlich machen sollen. Das heißt, das Teststreifensignal soll für Blut und Kontrolllösung bei gleichen Glukosekonzentrationen weitgehend gleich sein. EP-B 0 514 485 beschreibt ähnliche Kontrollflüssigkeiten, die Polystyrolsulfonat oder ein Salz davon als lösliches Polymer enthalten.

Aus US 5,679,573 ist es bekannt, in steroidhaltigen Kontrollflüssigkeiten durch den Zusatz von Cyclodextrinen die Steroidkomponente der Kontrolllösung zu stabilisieren. Ein Einfluss des Cydodextrins auf die Fließeigenschaften der Kontrolllösung ist nicht beschrieben.

In neuerer Zeit werden immer häufiger Testsysteme angeboten, bei denen die Probenflüssigkeit über eine Kapillare (Kapillarkanal, Kapillarspalt etc.) von einem Probenaufgabeort zu einem davon entfernten Messort auf dem Teststreifen transportiert wird. Entsprechende Teststreifen sind beispielsweise in WO 99/29429, WO 99/30158 oder WO 99/30152 beschrieben. Um Probenflüssigkeit, insbesondere Blut oder davon abgeleitete Körperflüssigkeiten (Plasma, Serum), schnell in die Kapillare des Teststriefens aufzunehmen, sind die Oberflächen der Kapillare oft mit einem Netzmittel behandelt.

Natürlich wird bei der Behandlung der Kapillarenoberfläche mit Netzmitteln versucht, ein möglichst optimales Füllverhalten für das Probenmaterial, beispielsweise also Blut, zu erreichen. Da die Kontrolllösungen sich in ihrem Fließverhalten von dem einer Blutprobe jedoch stark unterscheiden können, können bei der Benutzung von Kapillarspaltteststreifen zusammen mit Kontrollflüssigkeiten unerwünschte Effekte beobachtet werden: Die Kontrolllösung kann unter Umständen aus der Kapillare herausfließen und so beispielsweise Teile des Messgerätes, in welches der Teststreifen zwecks einer Messung eingelegt sein kann, verschmutzen oder gar zerstören, oder aus dem Teststreifen fließen oder tropfen und so die Umgebung des Teststreifens (beispielsweise Kleidung, Möbel etc.) verschmutzen.

Eine Möglichkeit, die geschilderten Probleme zu umgehen, bestünde darin, ähnlich wie in US 3,920,580 durch Zusatz eines "anti-diffusing agents" die Fließeigenschaft einer Kontrolllösung an die Fließeigenschaften von Blut anzugleichen. Insbesondere für Glucose als Analyt (und damit Glucose als wesentlichem Bestandteil der Kontrolllösung) ist dies jedoch nur bedingt möglich. Beispielsweise wird in Gegenwart von Albumin, das in US 3,920,580 als ein möglicher "anti-diffusing agent" empfohlen wird, in einer Kontrolllösung vorhandene Glucose nach und nach irreversibel zu glykiertem Albumin gebunden, so dass sich die Glucosekonzentration bei Lagerung der Kontrollflüssigkeit stetig verringert. Dies macht eine solche Kontrolllösung jedoch unbrauchbar, da es ja gerade für eine Kontrolllösung wichtig ist, eine definierte, konstante Konzentration des Zielanalyten für eine Messung bereitzustellen um so die Funktion des Messsystems zu überprüfen.

Im Stand der Technik fehlt es an Kontrolllösungen, mit denen das oben geschilderte Problem des unkontrollierten Fließens in einer Kapillare bei gleichzeitigem Erhalten einer konstanten Glucosekonzentration über längere (Lager-)Zeiträume gelöst ist. Es besteht daher der Bedarf nach Kontrollflüssigkeiten, die eine konstante Glucosekonzentration für einen längeren Zeitraum, typischerweise 2 Jahre, bereitstellen können und die bei der Verwendung zusammen mit Kapillarspaltteststreifen nicht zu einem unkontrollierten Fließverhalten neigen.

Die Aufgabe der vorliegenden Erfindung bestand darin, die Nachteile des Standes der Technik zu beseitigen. Insbesondere sollten Kontrollflüssigkeiten bereitgestellt werden, die eine konstante Glucosekonzentration für einen längeren Zeitraum, typischerweise 2 Jahre, bereitstellen können und die bei der Verwendung zusammen mit Kapillarspaltteststreifen nicht zu einem unkontrollierten Fließverhalten neigen.

Diese Aufgabe wird durch den Gegenstand der Erfindung, wie er in den Patentansprüchen charakterisiert ist, gelöst.

Gegenstand der Erfindung ist eine wässrige Kontrolllösung enthaltend Glucose in bekannter Konzentration und zumindest einen Stoff ausgewählt aus der Gruppe umfassend Cyclodextrine, Cyclodextrinderivate.

Die erfindungsgemäße Kontrollflüssigkeit oder Kontrolllösung ist im wesentlichen eine wässrige Lösung von Glucose. Vorzugsweise liegt die Glucose in vorgegebener, bekannter Konzentration in der Lösung vor. Dieser Lösung können übliche Zusatzstoffe wie Puffersubstanzen, Stabilisatoren, anorganische Salze und dergleichen mehr beigefügt sein. Bei der Auswahl der Zusatzstoffe ist lediglich darauf zu achten, daß die gewünschte Nachweisreaktion des Teststreifens nicht, insbesondere nicht negativ, durch sie beeinflußt wird. Für optische Nachweissysteme dürfen die Zusatzstoffe beispielsweise keinen Einfluß auf die Farbentwicklung der Indikatorsubstanz ausüben. Sinngemäßes gilt für elektrochemische Nachweissysteme oder enzymatische Reaktionen, die auf dem Teststreifen ablaufen.

Erfindungsgemäß enthält die Kontrollflüssigkeit zumindest einen Stoff ausgewählt aus der Gruppe umfassend Cyclodextrine (also α-, β- oder γ-Cyclodextrin) oder Cyclodextrinderivate, insbesondere ihre besser wasserlöslichen Hydroxypropylderivate, Obwohl nicht ganz geklärt ist, wie der Wirkmechanismus dieser Substanzen bei der Verwendung der erfindungsgemäßen Kontrolllösung ist, so beruht ihre Wirkung in der Kontrolllösung vermutlich darauf, dass sie in der Lage sind, Netzmittel; die in Teststreifen zum Erleichtern des Benetzens des Teststreifens durch Blutproben enthalten sind, zumindest teilweise zu binden und somit ihre Wirkung zumindest teilweise zu verhindern. Erwähnenswert erscheint auch die Tatsache, dass selbst hochviskose Lösungen, die in ein Kapillarspalttestelement nur langsam aufgenommen werden, ohne Zusatz der erfindungsgemäßeingesetzten Stoffe zu einem unkontrollierten Fließverhalten neigen und so potentiell Messgeräte und/oder Testelementumgebung verschmutzen können.

In einer bevorzugten Ausführungsform ist das Cyclodextrinderivat Hydroxypropyl-β-cyclodextrin. Dem Substitutionsgrad des Cyclodextrins kommt dabei keine besondere Bedeutung zu; insbesondere spielt er im bereich von 0,6 bis 1,0 praktisch keine Rolle. Hydroxypropyl-β-cyclodextrin hat sich als ganz besonders geeignet herausgestellt, weil es gut wasserlöslich ist, ohne die Viskosität der Kontrolllösung nennenswert zu erhöhen und damit die Füllzeit zu erhöhen, und in technischem Maßstab hergestellt wird. Hydroxypropyl-β-cyclodextrin ist z. B. als CAVASOL®W7 HP der Fa. Wacker Chemie GmbH, Burghausen, oder Cavitron 82006 der Fa. Cerestar, Krefeld, im Handel. Weitere Cyclodextrinderivate sind dem Fachmann an sich bekannt, beispielsweise aus V.J. Stella und R. A. Rajewski, Cyclodextrins: Their future in drug formulation and delivery, Pharm. Res. 14, 556- 567 (1997).

In einer alternativen Ausführungsform wird als fein dispergiertes Material mit großer spezifischer Oberfläche Aktivkohle oder Siliziumdioxid (beispielsweise bevorzugt in Form von Aerosil®) in der Kontrollflüssigkeit eingesetzt. Solche Kontrolllösungen neigen allerdings zur Sedimentation und sollten daher vor Gebrauch aufgeschüttelt werden. Aerosil® ist eine durch Hydrolyse von Siliziumtetrachlorid in einer Knallgasflamme hergestellte, hochdisperse "pyrogene" Kieselsäure von über 99,8% Siliziumdioxid-Gehalt.

Vorzugsweise enthält die erfindungsgemäße Kontrollflüssigkeit den Stoff, der aus der Gruppe umfassend Cyclodextrine, Cyclodextrinderivate und fein dispergierte Materialien mit großer spezifischer Oberfläche ausgewählt ist, in einer Konzentration von 5 mg/ml bis 50 mg/ml. Für Cyclodextrine oder deren Derivate haben sich Konzentrationen von 5 bis 50 mg/ml als bevorzugt erwiesen. Für fein dispergierte Materialien mit großer spezifischer Oberfläche wie z. B. Aktivkohle oder Siliziumdioxid (Aerosil) sind Konzentrationen von 5 bis 20 mg/ml bevorzugt.

Obwohl es bevorzugt ist, nur einen Stoff, der aus der Gruppe umfassend Cyclodextrine, Cyclodextrinderivate und fein dispergierte Materialien mit großer spezifischer Oberfläche ausgewählt ist, in der Kontrollflüssigkeit einzusetzen, ist es möglich, auch mehr als einen der genannten Stoffe einzusetzen.

Ein weiterer Gegenstand der Erfindung ist die Verwendung einer erfindungsgemäßen Kontrollflüssigkeit, wie sie weiter oben beschrieben wurde, zur Qualitäts- und/oder Funktionskontrolle eines Systems enthaltend Teststreifen und Messgerät, sowie ein Verfahren zur Funktionskontrolle eines Messsystems enthaltend Teststreifen und Messgerät für die Bestimmung von Glucose in flüssigen Proben umfassend das Kontaktieren des Teststreifens des Messsystems mit einer erfindungsgemäßen Kontrollflüssigkeit und das Erfassen des durch die in der Kontrollflüssigkeit enthaltenen Glucose verursachten Signals. Das Signal kann dabei eine Farbbildung oder Farbveränderung sein, die durch entsprechende Nachweisreagenzien auf dem Teststreifen im Zusammenwirken mit dem Analyten (Glucose) verursacht ist; in diesem Fall kann das Messgerät die Farbänderung, beispielsweise reflexionsphotometrisch, verfolgen. Das Signal kann aber auch die Änderung einer elektrischen Eigenschaft der Probe und/oder des Teststreifens sein, die vom Messgerät erfasst werden kann. Beispielsweise kann das Messsystem amperometrisch oder potentiometrisch arbeiten. All diese Möglichkeiten sind dem Fachmann bekannt.

Schließlich ist Gegenstand der Erfindung die Verwendung eines Stoffes ausgewählt aus der Gruppe umfassend Cyclodextrine, Cyclodextrinderivate und fein dispergierte Materialien mit großer spezifischer Oberfläche in einer Kontrollflüssigkeit (mit den oben genanten Bedeutungen und bevorzugten Formen) zum Binden eines Netzmittels aus einem mit Probe in Berührung kommenden Bereichs eines Testelements. Dabei kann das Binden des Netzmittels beispielsweise über Adsorption an die Oberfläche einer der genannten Substanzen erfolgen, wie dies beispielsweise für fein dispergierte Materialien mit großer spezifischer Oberfläche der Fall ist, oder über Einschluß des Netzmittels in entsprechende Stoffe, beispielsweise Cyclodextrine oder deren Derivate. Der genaue Wirkmechanismus ist für die vorliegende Erfindung von untergeordneter Bedeutung. Wichtig ist nur, dass das Netzmittel zumindest zum Teil gebunden und so quasi in seiner Funktion inaktiviert wird und so die Fließeigenschaften der Kontrolllösung an die einer "echten" Probe (z. B. Blut, in dem - wie sich im Verlauf der Untersuchungen zu der vorliegenden Erfindung herausgestellt hat - das Albumin für die Inaktivierung des Netzmittels verantwortlich sein könnte) angepasst werden. Die entsprechenden Bereiche des Testelements oder Teststreifens, die mit der Probe in Berührung kommen und die ein Netzmittel enthalten, können beispielsweise ein Kapillarkanal zum Probentransport, eine Spreitmittelschicht, eine Probenaufgabezone in Form eines saugfähigen Materials oder eines Näpfchens, eine reagenzienhaltige Nachweisschicht oder ähnliches sein. Dem Fachmann sind die Bereiche eines Teststreifens/Testelements bekannt, die mit der Probe (Probenflüssigkeit) in Berührung kommen und die Netzmittel enthalten können.

Die Erfindung wird durch die nachfolgenden Beispiele näher erläutert.

### Beispiel 1

### Glucosekontrollflüssigkeiten

Eine Hydroxypropyl-β-cyclodextrin-haltige Glucosekontrollflüssigkeit (=KF 1) wurde aus den in der nachfolgenden Tabelle 1 angegebenen Inhaltsstoffen zusammengemischt. Für Vergleichszwecke wurde eine Glucosekontrollflüssigkeit ohne Hydroxypropyl-β-cyclodextrin (Hydroxypropyl-β-CD) (=KF 2) hergestellt, die bis auf den Wasseranteil mit der Hydroxypropyl-β-cyclodextrin-haltigen Glucosekontrollflüssigkeit identisch war. Weitere erfindungsgemäße Kontrollflüssigkeiten konnten durch Dispergieren von 1,4 % Aerosil® 380 (= KF 3) bzw. 1 % Pulsorb®GW (= KF 4), beide von Degussa AG, Frankfurt/Main, in KF 2 erhalten werden.

**Tabelle 1**

| Inhaltsstoff | Menge [g] | | | |
|---|---|---|---|---|
| | KF 1 | KF 2 | KF 3 | KF 4 |
| bidestilliertes Wasser | 80,60 | 82,60 | 81,20 | 81,60 |
| KH₂PO₄ | 0,26 | 0,26 | 0,26 | 0,26 |
| Na₂HPO₄ | 0,54 | 0,54 | 0,54 | 0,54 |
| 2-Hydroxypyridin-N-oxid | 0,10 | 0,10 | 0,10 | 0,10 |
| Germal 115¹ | 0,10 | 0,10 | 0,10 | 0,10 |
| Kathon CG² | 0,10 | 0,10 | 0,10 | 0,10 |
| Glucose | 0,30 | 0,30 | 0,30 | 0,30 |
| Macrogol 6000³ | 3,50 | 3,50 | 3,50 | 3,50 |
| Glycerin | 12,50 | 12,50 | 12,50 | 12,50 |
| Hydroxypropyl-β-CD | 2,00 | 0 | 0 | 0 |
| Aerosil 380 | 0 | 0 | 1,40 | 0 |
| Pulsorb GW | 0 | 0 | 0 | 1,00 |
| Summe | 100 | 100 | 100 | 100 |

| | | | | |
|---|---|---|---|---|
| ¹ N,N"-Methylenbis-(N'-(hydroxymethyl)-2,6-dioxo-4-imidazolinylharnstoff) der Fa. ISP Glibal Technologies, Frechen | | | | |
| ² Gemisch aus 5-Chlor-2-methylisothiazol-3-on und 2-Methylisothiazol-3-on der Fa. C. H. Ebersloeh, Krefeld | | | | |
| ³ Polyethylenglykol der durchschnittlichen Molmasse 6000 der Fa. Clariant (Deutschland) GmbH, Frankfurt/Main | | | | |

### Beispiel 2

### Verwendung der Kontrollflüssigkeiten mit einem waagrecht abgelegten Kapillarspalttestelement

Jeweils 15 µl der Kontrollflüssigkeiten aus Beispiel 1 (KF 1und KF 2) wurden auf die Probenaufgabeöffnung eines waagrecht auf eine feste Unterlage abgelegten Kapillarspalttestelements, wie es in Figur 1 von WO 00/19185 beschrieben ist, aufgegeben. Die Kerbe der Probenaufgabeöffnung zeigte dabei nach oben. Die Kontrollflüssigkeit wurde durch Kapillarkräfte in den Kapillarspalt (Kapillarkanal) des Testelements aufgenommen, bis dieser gefüllt war. Der Rest der Kontrollflüssigkeit verblieb als kleiner Tropfen auf der Probenaufgabeöffnung. Es wurde beobachtet, in wievielen Fällen die aufgegebene Kontrollflüssigkeit nach 10 min aus dem der Probenaufgabeöffnung gegenüberliegenden Ende des Kapillarspalts (Entlüftungsöffnung) austrat. Wünschenwert ist dabei, dass keine Kontrollflüssigkeit aus der Entlüftungsöffnung austritt.

Mit der erfindungsgemäßen Kontrollflüssigkeit (KF 1 aus Beispiel 1) war in keinem der 51 untersuchten Fälle (entsprechend 0 %) nach 10 min ein Austritt der Kontrollflüssigkeit aus der Entlüftungsöffnung zu beobachten, das gleiche wurde mit KF3 und KF 4 beobachtet.

Mit der herkömmlichen Kontrollflüssigkeit (KF 2 aus Beispiel 1) war in 45 der 50 untersuchten Fälle (entsprechend 90 %) bereits nach 1 min ein Austritt der Kontrollflüssigkeit aus der Entlüftungsöffnung zu beobachten, nach 10 min in allen Fällen.

Anmerkung: Mit Blut als Probenflüssigkeit ist in keinem der Fälle ein Austritt aus der Entlüftungsöffnung zu beobachten.

### Beispiel 3

### Verwendung der Kontrollflüssigkeiten mit einem schräg gehaltenen Kapillarspalttestelement

Jeweils 15 µl der Kontrollflüssigkeiten aus Beispiel 1 (KF lund KF 2) wurden auf die Probenaufgabeöffnung eines waagrecht auf eine feste Unterlage abgelegten Kapillarspalttestelements, wie es in Figur 1 von WO 00/19185 beschrieben ist, aufgegeben. Die Kerbe der Probenaufgabeöffnung zeigte dabei nach oben. Die Kontrollflüssigkeit wurde durch Kapillarkräfte in den Kapillarspalt (Kapillarkanal) des Testelements aufgenommen, bis dieser gefüllt war. Der Rest der Kontrollflüssigkeit verblieb als kleiner Tropfen auf der Probenaufgabeöffnung. Danach wurde das Testelement gekippt, so dass die Probenaufgabeöffnung nach oben zeigte und das Testelement mit der waagrechten Unterlage einen Winkel von ca. 20 bis 25° einschloss. Es wurde beobachtet, in wievielen Fällen die aufgegebene Kontrollflüssigkeit nach 10 min aus dem der Probenaufgabeöffnung gegenüberliegenden Ende des Kapillarspalts (Entlüftungsöffnung) austrat. Wünschenwert ist dabei, dass keine Kontrollflüssigkeit aus der Entlüftungsöffnung austritt.

Mit der erfindungsgemäßen Kontrollflüssigkeit (KF 1 aus Beispiel 1) war in 6 der untersuchten 51 Fälle (entsprechend 12 %) nach 10 min ein Austritt der Kontrollflüssigkeit aus der Entlüftungsöffnung zu beobachten. Auch hier zeigten KF 3 und KF 4 ein ähnliches Verhalten, bei dem in 8 von 50 (= 16 %) bzw. 6 von 50 (= 12 %) Fällen ein Austritt beobachtet wurde.

Mit der herkömmlichen Kontrollflüssigkeit (KF 2 aus Beispiel 1) war in 50 der 50 untersuchten Fälle (entsprechend 100 %) bereits nach 1 min ein Austritt der Kontrollflüssigkeit aus der Entlüftungsöffnung zu beobachten.

Anmerkung: Mit Blut als Probenflüssigkeit ist in keinem der Fälle ein Austritt aus der Entlüftungsöffnung zu beobachten.

## Patentansprüche

1. Wässrige Kontrollflüssigkeit enthaltend Glucose in bekannter Konzentration und zumindest einen Stoff ausgewählt aus der Gruppe umfassend Cyclodextrine, Cyclodextrinderivate.

2. Kontrollflüssigkeit gemäß Anspruch 1, **dadurch gekennzeichnet, dass** das Cyclodextrinderivat Hydroxypropyl-β-cyclodextrin ist.

3. Kontrollflüssigkeit gemäß Anspruch 1, **dadurch gekennzeichnet, dass** der Stoff ausgewählt aus der Gruppe umfassend Cyclodextrine, Cydodextrinderivate in einer Konzentration von 5 mg/ml bis 50 mg/ml vorliegt.

4. Verwendung eines Stoffes ausgewählt aus der Gruppe umfassend Cyclodextrine, Cyclodextrinderivate und fein dispergierte Materialien mit großer spezifischer Oberfläche, sodass ein Netzmittel an die Oberfläche der Materialien binden kann, in einer Kontrollflüssigkeit zum Binden eines Netzmittels aus einem mit Probe in Berührung kommenden Bereichs eines Testelements.

5. Verwendung gemäß Anspruch 4, **dadurch gekennzeichnet, dass** das Cyclodextrinderivat Hydroxypropyl-β-cyclodextrin ist.

6. Verwendung gemäß Anspruch 4, **dadurch gekennzeichnet, dass** das fein dispergierte Material mit großer spezifischer Oberfläche Aktivkohle oder Siliziumdioxid ist.

7. Verwendung gemäß Anspruch 4, **dadurch gekennzeichnet, dass** der Stoff ausgewählt aus der Gruppe umfassend Cyclodextrine, Cyclodextrinderivate und fein dispergierte Materialien mit großer spezifischer Oberfläche in einer Konzentration von 5 mg/ml bis 50 mg/ml vorliegt.

8. Verwendung einer Kontrollflüssigkeit gemäß einem der Ansprüche 1 bis 3 zur Qualitäts- und/oder Funktionskontrolle eines Systems enthaltend Teststreifen und Messgerät.

9. Verfahren zur Funktionskontrolle eines Messsystems enthaltend Teststreifen und Messgerät für die Bestimmung von Glucose in flüssigen Proben umfassend das Kontaktieren des Teststreifens des Messsystems mit einer Kontrollflüssigkeit gemäß einem der Ansprüche 1 bis 3 und das Erfassen des durch die in der Kontrollflüssigkeit enthaltenen Glucose verursachten Signals.

## Claims

1. Aqueous control liquid containing glucose at a known concentration and at least one substance selected from the group comprising cyclodextrins, cyclodextrin derivatives.

2. Control liquid as claimed in claim 1, wherein the cyclodextrin derivative is hydroxypropyl-β-cyclodextrin.

3. Control liquid as claimed in claim 1, wherein the substance selected from the group comprising cyclodextrins, cyclodextrin derivatives at a concentration of 5 mg/ml to 50 mg/ml.

4. Use of a substance selected from the group comprising cyclodextrins, cyclodextrin derivatives and finely dispersed materials having a large specific surface so that a wetting agent can bind on the surface of the material in a control liquid to bind a wetting agent from a region of a test element that comes into contact with the sample.

5. Use as claimed in claim 4, wherein the cyclodextrin derivative is hydroxypropyl-β-cyclodextrin.

6. Use as claimed in claim 4, wherein the finely dispersed material having a large specific surface is active charcoal or silicon dioxide.

7. Use as claimed in claim 4, wherein the substance is selected from the group comprising cyclodextrins, cyclodextrin derivatives and finely dispersed materials having a large specific surface is present at a concentration of 5 mg/ml to 50 mg/ml.

8. Use of a control liquid as claimed in one of the claims 1 to 3 for the quality and/or functional control of a system comprising test strips and measuring instrument.

9. Method for controlling the function of a measuring system comprising test strips and measuring instrument for the determination of glucose in liquid samples comprising contacting the test strip of the measuring system with a control liquid as claimed in one of the claims 1 to 3 and detecting the signal caused by the glucose contained in the control liquid.

## Revendications

1. Liquide témoin aqueux contenant du glucose dans une concentration connue et au moins une substance choisie parmi le groupe comprenant des cyclodextrines, des dérivés de cyclodextrines.

2. Liquide témoin selon la revendication 1, **caractérisé en ce que** le dérivé de cyclodextrine est l'hydroxypropyl-β-cyclodextrine.

3. Liquide témoin selon la revendication 1, **caractérisé en ce que** la substance choisie parmi le groupe comprenant des cyclodextrines, des dérivés de cyctodextrines est présente en une concentration de 5 mg/ml à 50 mg/ml.

4. Utilisation d'une substance choisie parmi le groupe comprenant des cyclodextrines, des dérivés de cyclodextrines et des matériaux finement dispersés possédant une grande surface spécifique, de telle sorte qu'un agent mouillant peut venir se lier à la surface des matériaux, dans un liquide témoin pour la liaison d'un agent mouillant à partir d'une zone d'un élément d'essai, entrant en contact avec l'échantillon.

5. Utilisation selon la revendication 4, **caractérisée en ce que** le dérivé de cyclodextrine est l'hydroxypropyl-β-cydodextrine.

6. Utilisation selon la revendication 4, **caractérisée en ce que** le matériau finement dispersé possédant une grande surface spécifique représente du charbon actif ou du dioxyde de silicium.

7. Utilisation selon la revendication 4, **caractérisée en ce que** la substance choisie parmi le groupe comprenant des cyclodextrines, des dérivés de cyclodextrines et des matériaux finement dispersés possédant une grande surface spécifique est présente en une concentration de 5 mg/ml à 50 mg/ml.

8. Utilisation d'un liquide témoin selon l'une quelconque des revendications 1 à 3, pour le contrôle de la qualité et/ou pour le contrôle fonctionnel d'un système contenant une bandelette réactive et un appareil de mesure.

9. Procédé pour le contrôle fonctionnel d'un système de mesure contenant une bandelette réactive et un appareil de mesure pour la détermination de la teneur d'échantillons liquides en glucose, comprenant la mise en contact de la bandelette réactive du système de mesure avec un liquide témoin selon l'une quelconque des revendications 1 à 3 et l'enregistrement du signal dû au glucose contenu dans le liquide témoin.
